# EUROPÄISCHE PATENTSCHRIFT

(11) **EP 0 662 474 B1**
(45) Veröffentlichungstag und Bekanntmachung des Hinweises auf die Patenterteilung: **14.05.2003**
(21) Anmeldenummer: 94120769.8
(22) Anmeldetag: 27.12.1994
(51) Int. Cl.: C07D 213/61

(54) **VERFAHREN ZUR HERSTELLUNG VON CHLORPYRIDINEN**
PROCESS FOR THE PREPARATION OF CHLOROPYRIDINES
PROCEDE POUR LA PREPARATION DES CHLOROPYRIDINES

(30) Priorität: 11.01.1994 DE 4400462
(43) Veröffentlichungstag der Anmeldung: 12.07.1995
(73) Patentinhaber: Bayer CropScience AG, 40789 Monheim (DE)
(72) Erfinder: Lantzsch, Dr. Reinhard, D-42115 Wuppertal (DE); Jelich, Dr. Klaus, Overland Park, KS 66223 (US); Casser, Dr. Carl, D-51061 Köln (DE); Mannheims, Dipl.-Ing. Christoph, D-51373 Leverkusen (DE); Lawrenz, Dr. Knud, D-51067 Köln (DE)

(56) Entgegenhaltungen:
- EP-A- 0 072 777
- EP-A- 0 546 418
- JOURNAL OF MEDICINAL CHEMISTRY, Bd.33, 1990, WASHINGTON US Seiten 1667 - 1675 WAYNE K. ANDERSON ET AL. 'Synthesis, chemistry, and antineoplastic activity of alpha-halopyridinium salts: ...'
- JOURNAL OF ORGANIC CHEMISTRY., Bd.24, 1959, EASTON US Seiten 756 - 760 JEROME A. BERSON ET AL. 'Synthetic approaches to Ipeac Alkaloids. III: ...'
- 'The Chemistry of Heterocyclic Compounds. Pyridine and its Derivatives, Part II, p. 20', 1960, WILEY, NEW YORK
- J. AM. CHEM. SOC. Bd. 75, 1953, Seiten 4482 - 4484
- J. AM. CHEM. SOC. Bd. 78, 1956, Seiten 416 - 418

## Beschreibung

Die Erfindung betrifft ein neues Verfahren zur Herstellung von Chlorpyridinen, welche als Zwischenprodukte zur Herstellung von agrochemischen oder pharmazeutischen Wirkstoffen verwendet werden können, sowie neue Chlorpyridiniumchloride und ein Verfahren zu ihrer Herstellung.

Es ist bekannt, dass durch Umsetzung von bestimmten Acetenamiden mit Phosphorylchlorid/Dimethylformamid 5-substituierte 2-Chlor-pyridine hergestellt werden können (vgl. EP-A 0 546 418).

Weiter ist bekannt, dass man Chlorpyridiniumbromide erhält, wenn man Pyridine mit Benzylbromiden umsetzt (vgl. Chem. Ber. 103 (1970), 3427-3436; J. Heterocyclic Chem. 28 (1991), 1083-1089). Hierfür sind jedoch lange Umsetzungszeiten - im allgemeinen mehrere Tage - erforderlich, und die Ausbeuten sind meist unbefriedigend.

Ferner ist bekannt, dass Chlorpyridiniumchloride durch Umsetzung von N-Alkylpyridonen mit Phosphor(V)-chlorid hergestellt werden können (vgl. US-P 3,149,105, Example 3). Hierbei werden jedoch die Produkte in unbefriedigender Qualität und in mäßiger Ausbeute erhalten. Pyridone mit dem gewünschten Substitutionsmuster sind zudem nur schwierig herstellbar.

Gegenstand der vorliegenden Erfindung sind
(1) ein Verfahren zur Herstellung der Chlorpyridine der Formel (IV) 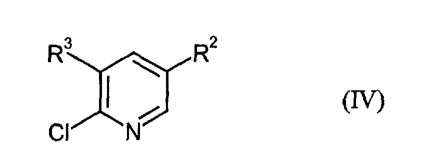 in welcher
   - R²: für jeweils gegebenenfalls durch Fluor, Chlor, Brom, Methoxy oder Ethoxy substituiertes Methyl, Ethyl, n- oder i-Propyl, n-, i-, s- oder t-Butyl steht und
   - R³: für Wasserstoff, Chlor oder für jeweils gegebenenfalls durch Fluor oder Chlor substituiertes Methyl, Ethyl, n- oder i-Propyl, n-, i-, s- oder t-Butyl steht,
   dadurch gekennzeichnet, dass man in einem ersten Schritt Enamide der allgemeinen Formel (II) 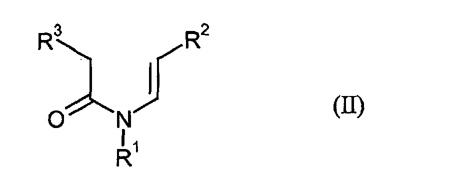 in welcher
   - R¹: für Methyl, Ethyl, n- oder i-Propyl, n-, i-, s- oder t-Butyl, Propenyl, Butenyl, Propinyl, Butinyl oder für jeweils gegebenenfalls durch Chlor, Methyl, Ethyl, n- oder i-Propyl, n-, i-, s- oder t-Butyl substituiertes Benzyl, 1-Phenyl-ethyl oder 2-Phenyl-ethyl steht und
   - R² und R³: die oben angegebene Bedeutung haben,
   mit einem Chlorierungsmittel in Gegenwart eines Formamid-Derivates der allgemeinen Formel (III) 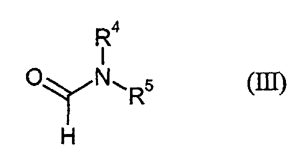 in welcher
   - R⁴ und R⁵: einzeln für Methyl, Ethyl, n- oder i-Propyl, n-, i-, s- oder t-Butyl, Cyclopentyl oder Cyclohexyl, oder zusammen für Butan-1,4-diyl oder Pentan-1,5-diyl stehen,
   bei Temperaturen in der Anfangsphase zwischen -10°C und +40°C und anschließend zwischen +40°C und +75°C gegebenenfalls in Gegenwart eines Verdünnungsmittels umsetzt und die so erhaltenen Chlorpyridiniumchloride der Formel (I) 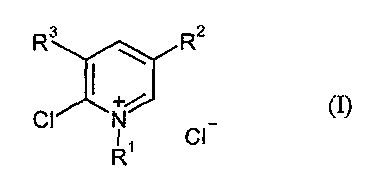 in welcher
   R¹, R² und R³ die oben angegebene Bedeutung haben,
   nach deren Isolierung in einer zweiten Stufe im Temperaturbereich zwischen 75°C und 200°C thermisch spaltet;
(2) neue Chlorpyridiniumchloride der allgemeinen Formel (I) 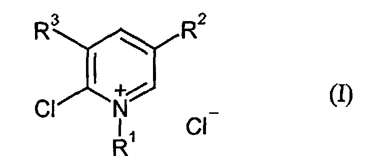 in welcher
   - R¹: für Methyl, Ethyl, n- oder i-Propyl, n-, i-, s- oder t-Butyl, Propenyl, Butenyl, Propinyl, Butinyl oder für jeweils gegebenenfalls durch Chlor, Methyl, Ethyl, n- oder i-Propyl, n-, i-, s- oder t-Butyl substituiertes Benzyl, 1-Phenyl-ethyl oder 2-Phenyl-ethyl steht,

   - R²: für jeweils gegebenenfalls durch Fluor, Chlor, Brom, Methoxy oder Ethoxy substituiertes Methyl, Ethyl, n- oder i-Propyl, n-, i-, s- oder t-Butyl steht und
   - R³: für Wasserstoff, Chlor oder für jeweils gegebenenfalls durch Fluor oder Chlor substituiertes Methyl, Ethyl, n- oder i-Propyl, n-, i-, s- oder t-Butyl steht,
   ausgenommen die Verbindung, in welcher R¹ für 2-Phenyl-ethyl, R² für Ethyl und R³ für Wasserstoff steht;
(3) ein Verfahren zur Herstellung von Chlorpyridiniumchloriden der allgemeinen Formel (I), dadurch gekennzeichnet, dass man Enamide der allgemeinen Formel (II) 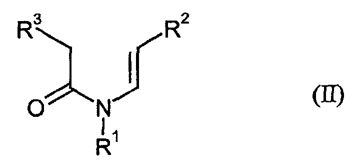
in welcher
R¹, R² und R³ die oben angegebene Bedeutung haben,
mit einem Chlorierungsmittel in Gegenwart eines Formamid-Derivates der allgemeinen Formel (III) 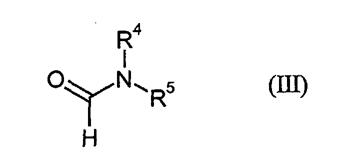 in welcher
R⁴ und R⁵ die oben angegebene Bedeutung haben,
bei Temperaturen in der Anfangsphase zwischen -10°C und +40°C und anschließend zwischen +40°C und +75°C gegebenenfalls in Gegenwart eines Verdünnungsmittels umsetzt.

Überraschenderweise können die neuen Chlorpyridiniumchloride der Formel (I) nach dem erfindungsgemäßen Verfahren (3) auf einfachere Weise und in wesentlich besseren Ausbeuten als nach den bekannten Methoden erhalten werden.

Das erfindungsgemäße Verfahren (3) erweist sich im Vergleich mit vorbekannten Methoden auch als ein wesentlich kostengünstigerer Herstellungsweg für Chlorpyridiniumchloride der allgemeinen Formel (I) und stellt somit eine wertvolle Bereicherung des Standes der Technik dar. Chlorpyridiniumchloride der Formel (I) dienen zur Herstellung der entsprechenden Chlorpyridine.

Verwendet man beispielsweise N-Ethyl-N-(1-propen-1-yl)-acetamid, Phosgen und N,N-Dimethylformamid als Ausgangsstoffe, so kann der Reaktionsablauf beim erfmdungsgemäßen Verfahren (3) bzw. beim ersten Schritt des erfindungsgemäßen Verfahrens (1) durch das folgende Formelschema skizziert werden:

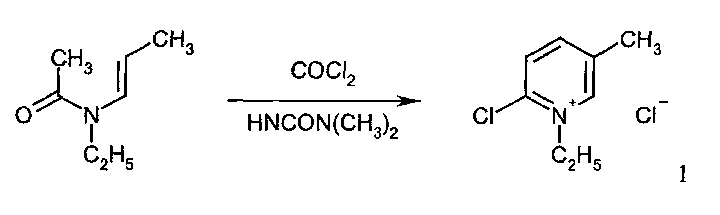

Die beim erfindungsgemäßen Verfahren (3) bzw. beim ersten Schritt des erfindungsgemäßen Verfahrens (1) zur Herstellung der Verbindungen der allgemeinen Formel (I) als Ausgangsstoffe zu verwendenden Enamide sind durch die Formel (II) allgemein definiert. In der Formel (II) haben R¹, R² und R³ diejenigen Bedeutungen, die bereits oben im Zusammenhang mit der Beschreibung der neuen Verbindungen der Formel (I) angegeben wurden.

Die Ausgangsstoffe der allgemeinen Formel (II) sind bekannt und/oder können nach an sich bekannten Verfahren hergestellt werden (vgl. EP-A 546418; J. Chem. Soc. Perkin Trans. I 1984, 1173-1182).

Das erfindungsgemäße Verfahren (3) bzw. der erste Schritt des erfindungsgemäßen Verfahrens (1) wird unter Verwendung eines Chlorierungsmittels durchgeführt. Es können hierbei die üblichen Chlorierungsmittel verwendet werden, beispielsweise Phosphorylchlorid (Phosphoroxychlorid), Phosphor(V)-chlorid, Phosgen, Oxalylchlorid, Thionylchlorid, Perchlorbutansäurechlorid, Dichlorbenzodioxol, N,N-Dimethyl-chlormethylimmoniumchlorid oder N,N-Diethyl-chlormethylimmoniumchlorid.

Phosgen wird als Chlorierungsmittel beim erfindungsgemäßen Verfahren (3) bzw. beim ersten Schritt des erfindungsgemäßen Verfahrens (1) besonders bevorzugt.

Die beim erfindungsgemäßen Verfahren (3) bzw. beim ersten Schritt des erfindungsgemäßen Verfahrens (1) zur Herstellung der Verbindungen der allgemeinen Formel (I) weiter zu verwendenden Formamid-Derivate sind durch die Formel (III) allgemein definiert. In der Formel (III) stehen R⁴ und R⁵ einzeln für Methyl, Ethyl, n-oder i-Propyl, n-, i-, s- oder t-Butyl, Cyclopentyl oder Cyclohexyl, oder zusammen für Butan-1,4-diyl oder Pentan-1,5-diyl.

### Als Beispiele für die Formamid-Derivate der Formel (III) seien genannt:

N,N-Dimethyl-formamid, N,N-Diethyl-formamid, N,N-Dipropyl-formamid, N,N-Dibutyl-formamid, N-Cyclohexyl-N-methyl-formamid, N,N-Dicyclohexyl-formamid.

Als besonders bevorzugte Formamid-Derivate seien N,N-Dimethyl-formamid und N,N-Dibutyl-formamid genannt.

Die Formamid-Derivate der Formel (III) sind bekannte organische Synthesechemikalien.

Als Verdünnungsmittel zur Durchführung des erfindungsgemäßen Verfahrens (3) bzw. des ersten Schrittes des erfindungsgemäßen Verfahrens (1) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Nitrile, wie Acetonitril, Propionitril oder Butyronitril; Amide, wie N,N-Dimethylformamid, N,N-Dimethylacetamid, N-Methylformanilid, N-Methyl-pyrrolidon oder Hexamethylphosphorsäuretriamid; Sulfoxide, wie Dimethylsulfoxid, sowie Sulfone, wie Tetramethylensulfon.

Als besonders bevorzugte Verdünnungsmittel seien Chlorbenzol und Acetonitril genannt.

Die Reaktionstemperaturen können bei der Durchführung des erfindungsgemäßen Verfahrens (3) bzw. des ersten Schrittes des erfindungsgemäßen Verfahrens (1) in einem größeren Bereich variiert werden. Im allgemeinen arbeitet man bei Temperaturen in der Anfangsphase zwischen -10°C und +40°C und anschließend zwischen +40°C und +75°C.

Das erfindungsgemäße Verfahren (3) bzw. der erste Schritt des erfindungsgemäßen Verfahrens (1) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (3) bzw. des ersten Schrittes des erfindungsgemäßen Verfahrens (1) zur Herstellung der Verbindungen der Formel (I) setzt man pro Mol an Enamid der Formel (II) im allgemeinen zwischen 1 und 10 Mol, vorzugsweise zwischen 1,5 bis 5,0 Mol, insbesondere zwischen 2,0 und 3,0 Mol Chlorierungsmittel und zwischen 1 und 10 Mol, vorzugsweise zwischen 1,0 und 2,0 Mol Formamid-Derivat der Formel (III) ein.

Die Reaktionskomponenten können bei der Durchführung des erfindungsgemäßen Verfahrens (3) bzw. des ersten Schrittes des erfindungsgemäßen Verfahrens (1) in beliebiger Reihenfolge mit einander umgesetzt werden.

In einer bevorzugten Ausführungsform des erfindungsgemäßen Verfahrens (3) bzw. des ersten Schrittes des erfindungsgemäßen Verfahrens (1) werden zunächst das Formamid-Derivat der Formel (III) und das Chlorierungsmittel in einem Verdünnungsmittel vorgelegt und das Enamid der Formel (II) wird dann bei Temperaturen zwischen -10°C und +50°C zudosiert; anschließend wird erneut Chlorierungsmittel eindosiert. Man führt dann die Umsetzung durch ein- oder mehrstündiges Rühren bei etwas erhöhter Temperatur zu Ende.

Die Aufarbeitung kann auf übliche Weise durchgeführt werden. Beispielsweise wird mit Wasser auf das zwei- bis dreifache Volumen verdünnt. Die wässrige Phase wird durch Zugabe einer Base, wie z.B. Natronlauge, auf einen pH-Wert zwischen 2 und 7 eingestellt und dann wird das Lösungsmittel (Wasser) unter vermindertem Druck sorgfältig abdestilliert. Das als Rückstand verbleibende Rohprodukt kann auf übliche Weise weiter gereinigt werden; es kann aber auch als solches zu weiteren Umsetzungen verwendet werden.

Die nach dem erfindungsgemäßen Verfahren (3) herstellbaren Chlorpyridiniumchloride der Formel (I) können als Zwischenprodukte zur Herstellung von agrochemischen oder pharmazeutischen Wirkstoffen - bzw. zur Herstellung von deren Vorprodukten (vgl. die Herstellungsbeispiele) - verwendet werden (erfindungsgemäßes Verfahren (1)); zum Beispiel erhält man durch thermische Spaltung die Chlorpyridine der Formel (IV) 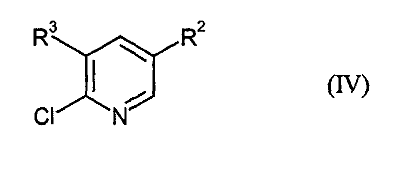 und Chloride der Formel R¹-Cl. Je nach Substituent R¹ sind für die Thermolyse verschiedene Temperaturbereiche notwendig, im allgemeinen zwischen 75°C und 200°C. Die beiden Komponenten können durch Destillation getrennt werden.

### Herstellungsbeispiele:

### Beispiel 1

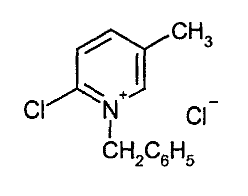

In 800 ml Chlorbenzol werden 345,4 g (2,2 Mol) N,N-Dibutyl-formamid vorgelegt. 396 g (4 Mol) Phosgen werden eingeleitet, das Gemisch auf 40°C erwärmt und bei einer Innentemperatur von 35°C - 40°C 388 g (2 Mol) N-Benzyl-N-(l-propen-1-yl)-acetamid (Gehalt: 97,4%) zugetropft. Bei 45°C Innentemperatur werden weitere 99 g (0,5 Mol) Phosgen eingeleitet. Das Gemisch wird auf 70°C erwärmt und 1 Stunde bei 70°C gerührt. Anschließend wird auf Raumtemperatur (20°C) abgekühlt, 500 ml Wasser zugegeben und durch Zutropfen von ca. 140 g (1,6 Mol) konz. Natronlauge ein pH Wert von 5 eingestellt (Kühlung, Innentemperatur max. 30°C). Die Phasen werden getrennt und die wäßrige Phase bei max. 50°C Innentemperatur bis zur Trockene eingedampft.Man erhält 590 g eines hellbeigen Feststoffs, der laut HPLC und ¹H-NMR zu 65% aus N-Benzyl-2-chlor-5-methyl-pyridiniumchlorid besteht = 75,5% der Theorie.

Durch Verrühren mit Essigester erhält man das reine Salz (Schmp. 71°C-74°C).
¹H-NMR (D₂O): 2,5 ppm (s; CH₃), 5,9 ppm (s; CH₃), 7,4 und 7,5 ppm (m, C₆H₅), 8,07 und 8,1 (d, CH), 8,36 und 8,39 (dd, CH), 8,8 (s, CH).

### Beispiel 2

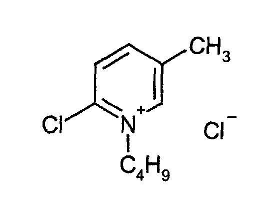

15,5 g (0,1 Mol) N-Butyl-N-(1-propen-1-yl)-acetamid und 8 g (0,11 Mol) Dimethylformamid werden in 80 ml trockenem Acetonitril gelöst und unter Rühren 25,4 g (0,2 Mol) Oxalylchlorid innerhalb von 30 Minuten bei 15°C getropft. Dann wird das Reaktionsgemisch auf 40°C erhitzt und nochmals 12,7 g (0,1 Mol) Oxalylchlorid innerhalb von 15 Minuten zugetropft. Anschließend rührt man 10 Stunden bei 40°C bis 45°C nach. Man destilliert das Acetonitril im Vakuum ab und rührt das zurückbleibende Öl in 350 ml Wasser ein, stellt mit Natronlauge auf pH 5 und extrahiert dreimal mit Methylenchlorid. Die Wasserphase wird im Vakuum zur Trockene eingedampft. Der Rückstand besteht hauptsächlich aus 2-Chlor-5-methyl-N-butyl-pyridiniumchlorid, das beim Stehen langsam zu kristallisieren beginnt (Schmp. 132°C bis 136°C (Z.)).Analog zu den Beispielen 1 und 2 sowie entsprechend der allgemeinen Beschreibung des erfindungsgemäßen Herstellungsverfahrens können beispielsweise die in der nachstehenden Tabelle 1 aufgeführten Verbindungen der Formel (I) hergestellt werden.

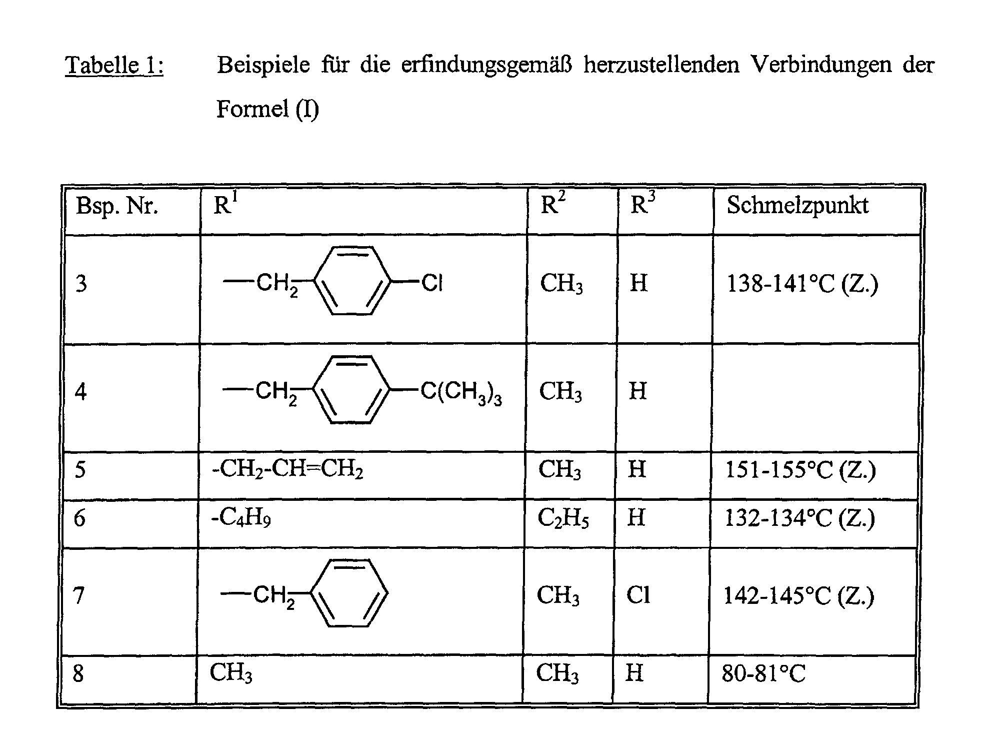

### Beispiele für die Umwandlung zu Chlorpyridinen der Formel (IV):

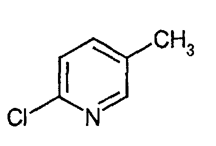

25,4 g (0,1 Mol) N-Benzyl-2-chlor-5-methyl-pyridiniumchlorid werden in einer Destillationsapparatur auf 80°C - 140°C/Wasserstrahlvakuum erhitzt. Es destilliert ein Gemisch aus 2-Chlor-5-methyl-pyridin und Benzylchlorid über, die durch eine Feindestillation getrennt werden können. Die Ausbeute ist praktisch quantitativ.

Die Zersetzungstemperatur von N-n-Butyl-2-chlor-5-methyl-pyridiniumchlorid liegt zwischen 130°C und 160°C, von N-Propenyl-2-chlor-5-methyl-pyridiniumchlorid zwischen 145°C und 180°C. Die Ausbeuten an 2-Chlor-5-methyl-pyridin sind ebenfalls quantitativ.

## Patentansprüche

1. Verfahren zur Herstellung der Chlorpyridine der Formel (IV) 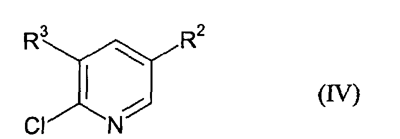 in welcher
R² für jeweils gegebenenfalls durch Fluor, Chlor, Brom, Methoxy oder Ethoxy substituiertes Methyl, Ethyl, n- oder i-Propyl, n-, i-, s- oder t-Butyl steht und
R³ für Wasserstoff, Chlor oder für jeweils gegebenenfalls durch Fluor oder Chlor substituiertes Methyl, Ethyl, n- oder i-Propyl, n-, i-, s- oder t-Butyl steht,
**dadurch gekennzeichnet, dass** man in einem ersten Schritt Enamide der allgemeinen Formel (II) 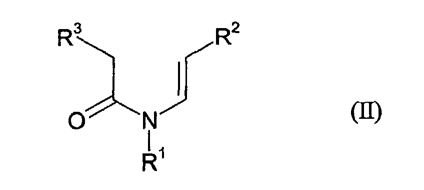 in welcher
R¹ für Methyl, Ethyl, n- oder i-Propyl, n-, i-, s- oder t-Butyl, Propenyl, Butenyl, Propinyl, Butinyl oder für jeweils gegebenenfalls durch Chlor, Methyl, Ethyl, n- oder i-Propyl, n-, i-, s- oder t-Butyl substituiertes Benzyl, 1-Phenyl-ethyl oder 2-Phenyl-ethyl steht und
R² und R³ die oben angegebene Bedeutung haben,
mit einem Chlorierungsmittel in Gegenwart eines Formamid-Derivates der allgemeinen Formel (III) 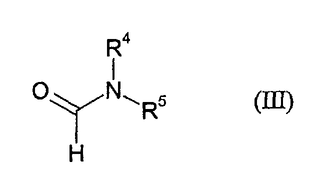 in welcher
R⁴ und R⁵ einzeln für Methyl, Ethyl, n- oder i-Propyl, n-, i-, s- oder t-Butyl, Cyclopentyl oder Cyclohexyl, oder zusammen für Butan-1,4-diyl oder Pentan-1,5-diyl stehen,
bei Temperaturen in der Anfangsphase zwischen -10°C und +40°C und anschließend zwischen +40°C und +75°C gegebenenfalls in Gegenwart eines Verdünnungsmittels umsetzt und die so erhaltenen Chlorpyridiniumchloride der Formel (I) 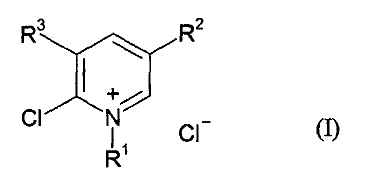 in welcher
R¹, R² und R³ die oben angegebene Bedeutung haben,
nach deren Isolierung in einer zweiten Stufe im Temperaturbereich zwischen 75°C und 200°C thermisch spaltet.

2. Chlorpyridiniumchloride der allgemeinen Formel (I) gemäß Anspruch 1, 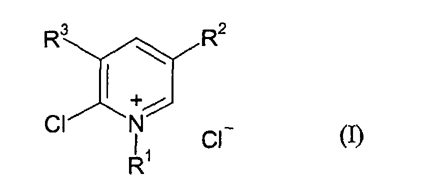 in welcher
R¹ für Methyl, Ethyl, n- oder i-Propyl, n-, i-, s- oder t-Butyl, Propenyl, Butenyl, Propinyl, Butinyl oder für jeweils gegebenenfalls durch Chlor, Methyl, Ethyl, n- oder i-Propyl, n-, i-, s- oder t-Butyl substituiertes Benzyl, 1-Phenyl-ethyl oder 2-Phenyl-ethyl steht,
R² für jeweils gegebenenfalls durch Fluor, Chlor, Brom, Methoxy oder Ethoxy substituiertes Methyl, Ethyl, n- oder i-Propyl, n-, i-, s- oder t-Butyl steht und
R³ für Wasserstoff, Chlor oder für jeweils gegebenenfalls durch Fluor oder Chlor substituiertes Methyl, Ethyl, n- oder i-Propyl, n-, i-, s- oder t-Butyl steht,
ausgenommen die Verbindung, in welcher R¹ für 2-Phenyl-ethyl, R² für Ethyl und R³ für Wasserstoff steht.

3. Verfahren zur Herstellung von Chlorpyridiniumchloriden der allgemeinen Formel (I) gemäß Anspruch 1, **dadurch gekennzeichnet, dass** man Enamide der allgemeinen Formel (II) 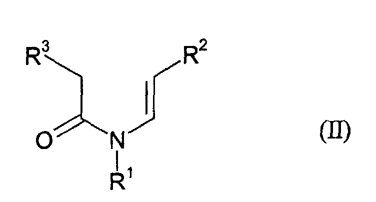 in welcher
R¹, R² und R³ die in Anspruch 1 angegebene Bedeutung haben,
mit einem Chlorierungsmittel in Gegenwart eines Formamid-Derivates der allgemeinen Formel (III) 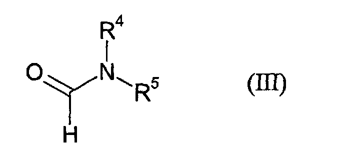 in welcher
R⁴ und R⁵ die in Anspruch 1 angegebene Bedeutung haben,
bei Temperaturen in der Anfangsphase zwischen -10°C und +40°C und anschließend zwischen +40°C und +75°C gegebenenfalls in Gegenwart eines Verdünnungsmittels umsetzt.

## Claims

1. Process for the preparation of chloropyridines of the formula (IV) 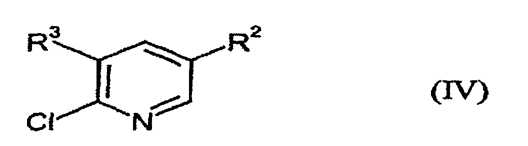 in which
R² represents in each case optionally fluorine-, chlorine-, bromine-, methoxy- or ethoxy-substituted methyl, ethyl, n- or i-propyl, n-, i-, s-or t-butyl and
R³ represents hydrogen, chlorine or represents in each case optionally fluorine- or chlorine-substituted methyl, ethyl, n- or i-propyl, n-, i-, s-or t-butyl,
**characterized in that** in a first step enamides of the general formula (II) 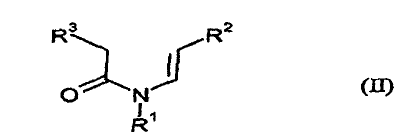 in which
R¹ represents methyl, ethyl, n- or i-propyl, n-, i-, s- or t-butyl, propenyl, butenyl, propynyl or butynyl or represents in each case optionally chlorine-, methyl-, ethyl-, n- or i-propyl-, n-, i-, s- or t-butyl-substituted benzyl, 1-phenyl-ethyl or 2-phenyl-ethyl and
R² and R³ are as defined above
are reacted with a chlorinating agent in the presence of a formamide derivative of the general formula (III) 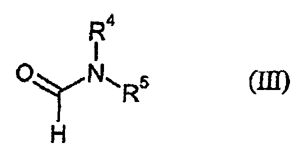 in which
R⁴ and R⁵ individually represent methyl, ethyl, n- or i-propyl, n-, i-, s- or t-butyl, cyclopentyl or cyclohexyl or together represent butane-1,4-diyl or pentane-1,5-diyl
at temperatures in the initial phase of between -10°C and +40°C and subsequently between +40°C and +75°C, optionally in the presence of a diluent, and in a second stage thermal cleavage of the resulting chloropyridinium chlorides of the formula (I) 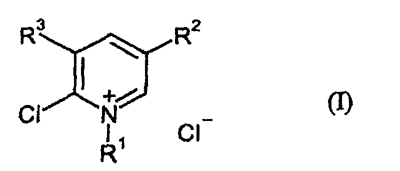 in which
R¹, R² and R³are as defined above,
following their isolation, is carried out in the temperature range between 75°C and 200°C.

2. Chloropyridinium chlorides of the general formula (I) according to Claim 1 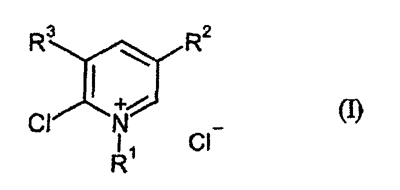 in which
R¹ represents methyl, ethyl, n- or i-propyl, n-, i-, s- or t-butyl, propenyl, butenyl, propynyl or butynyl or represents in each case optionally chlorine-, methyl-, ethyl-, n- or i-propyl-, n-, i-, s- or t-butyl-substituted benzyl, 1-phenyl-ethyl or 2-phenyl-ethyl,
R² represents in each case optionally fluorine-, chlorine-, bromine-, methoxy- or ethoxy-substituted methyl, ethyl, n- or i-propyl, n-, i-, s-or t-butyl and
R³ represents hydrogen, chlorine or represents in each case optionally fluorine- or chlorine-substituted methyl, ethyl, n- or i-propyl, n-, i-, s-or t-butyl,
with the exception of the compound in which R² represents 2-phenyl-ethyl, R² represents ethyl and R³ represents hydrogen.

3. Process for the preparation of chloropyridinium chlorides of the general formula (I) according to Claim 1, **characterized in that** enamides of the general formula (II) 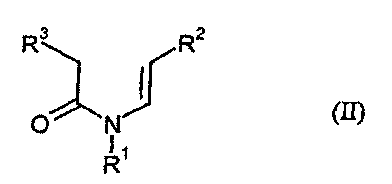 in which
R¹, R² and R³ are as defined in Claim 1
are reacted with a chlorinating agent in the presence of a formamide derivative of the general formula (III) 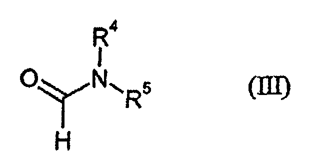 in which
R⁴ and R⁵ are as defined in Claim 1
at temperatures in the initial phase of between -10°C and +40°C and subsequently between +40°C and +75°C, optionally in the presence of a diluent.

## Revendications

1. Procédé de préparation des chloropyridines de formule (IV) : 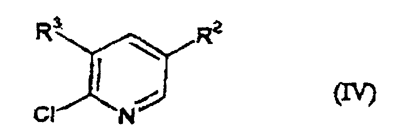 dans laquelle
R² représente méthyle, éthyle, n- ou i-propyle, n-, i-, s- ou t-butyle, le cas échéant substitué par fluor, chlore, brome, méthoxy ou éthoxy, et
R³ est hydrogène, chlore ou méthyle, éthyle, n- ou i-propyle, n-, i- s- ou t-butyle, le cas échéant substitué par fluor ou chlore,
**caractérisé en ce que** l'on a fait réagir dans une première étape des énamides de formule générale (II) 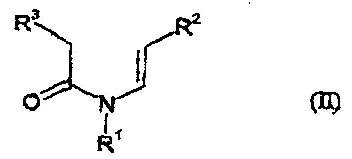 dans laquelle :
R¹ est méthyle, éthyle, n- ou i-propyle, n-, i-, sou t-butyle, propényle, butényle, propinyle, butinyle ou benzyle, 1-phényl-éthyle ou 2-phényl-éthyle, le cas échéant substitué par chlore, méthyle, éthyle, n- ou i-propyle, n-, i-, s- ou t-butyle, et
R² et R³ ont la signification indiquée ci-dessus, avec un agent de chloration en présence d'un dérivé de la formamide de formule générale (III) 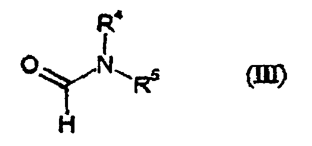
dans laquelle :
R⁴ et R⁵ sont individuellement méthyle, éthyle, n-ou i-propyle, n-, i-, s-, ou t-butyle, cyclopentyle ou cyclohexyle, ou sont ensemble butan-1,4-diyle ou pentan-1,5-diyle,
à des températures dans la phase initiale entre - 10°C et +40°C et ensuite entre +40°C et +75°C, le cas échéant en présence d'un diluant, et que l'on coupe par voie thermique les chlorures de chloropyridinium ainsi obtenus de formule (I) 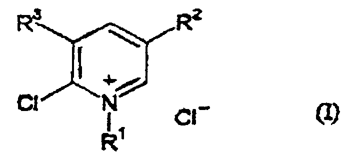 dans laquelle
R¹, R² et R³ ont la signification ci-dessus indiquée après leur isolement dans une deuxième étape, dans une plage de température entre 75°C et 200°C.

2. Chlorures de chloropyridinium de formule générale (I) selon la revendication (1) 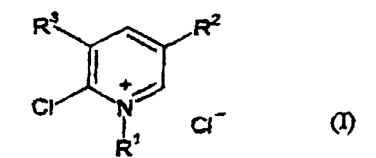 dans laquelle :
R¹ est méthyle, éthyle, n- ou i-propyle, n-, i-, sou t-butyle, propényle, butényle, propinyle, butinyle ou benzyle, 1-phényl-éthyle ou 2-phényl-éthyle, le cas échéant substitué par chlore, méthyle, éthyle, n- ou i-propyle, n-, i-, s- ou t-butyle,
R² est méthyle, éthyle, n- ou i-propyle, n-, i-, s-, ou t-butyle, le cas échéant substitué par fluor, chlore, brome, méthoxy ou éthoxy, et
R³ est hydrogène, chlore ou méthyle, éthyle, n- ou i-propyle, n-, i, s- ou t-butyle, le cas échéant substitué par fluor ou chlore,
à l'exception de la combinaison dans laquelle R¹ est phényl-éthyle, R² est éthyle et R³ est hydrogène.

3. Procédé de préparation de chlorures de chloropyridinium de formule générale (I), selon la revendication 1, **caractérisé en ce que** l'on fait réagir des énamides de formule générale (II) 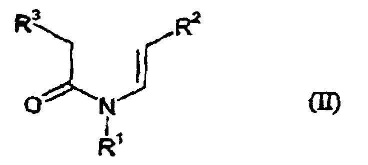 dans laquelle
R¹, R² et R³ ont la signification indiquée dans la revendication 1,
avec un agent de chloration en présence d'un dérivé de la formamide de formule générale (III) 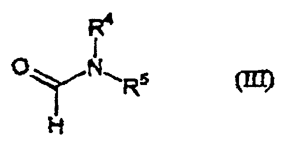 dans laquelle
R⁴ et R⁵ ont la signification indiquée dans la revendication 1.
à des températures dans la phase initiale se situant entre -10°C et +40°C et ensuite entre +40°C et +75°C, le cas échéant en présence d'un diluant.
